# EUROPEAN PATENT SPECIFICATION

(11) **EP 1 721 767 B1**
(45) Date of publication and mention of the grant of the patent: **30.04.2008**
(21) Application number: 06075542.8
(22) Date of filing: 08.03.2006
(51) Int. Cl.: B60H 3/00

(54) **Air freshener for vehicles**
Duftspender für Fahrzeuge
Désodorisant pour véhicule

(30) Priority: 12.05.2005 IT RE20050049
(43) Date of publication of application: 15.11.2006
(73) Proprietor: RE.LE.VI. S.p.a., 46040 Rodigo (Mantova) (IT)
(72) Inventor: Pagani, Fabio, c/o RE.LE.VI. - S.P.A., I-46040 Rodigo (Mantova) (IT)
(74) Representative: Corradini, Corrado

(56) References cited:
- EP-A- 1 319 543
- WO-A-20/05056063
- DE-U1- 20 207 512

## Description

The present invention concerns an air freshener for vehicles and, more specifically an air freshener for vehicles intended to be applied to one of the air vents of the air conditioning system of the vehicle itself, so as to exploit the air coming out from it as the vehicle for spreading a fragrance.

As known, air fresheners for vehicles of this type generally comprise an air freshening element that releases said fragrance, and a diffuser suitable for containing the air freshening element, which is provided with a claw that is fixed to the air vent.

Said diffuser usually has a group of aeration openings, through which the air coming out from the vent hits the air freshening element, taking the fragrance with it and spreading it around in the driving compartment; moreover, it is equipped with suitable adjustment means that, by adjustably closing said aeration openings, allow the flow rate of the air going in, and consequently the intensity of the air freshener, to be adjusted.

Currently, there are many models of air freshener for vehicles made in accordance with this constructive scheme on the market, which differ from each other in terms of the shape of the diffuser, the operation of the adjustment means, and also the type of air freshening element used.

In DE 202 07 512 U, a diffuser according to the preamble of claim 1 is shown having a diffuser shell suitable for enclosing air freshening material, which is arranged to be applied to one of the vehicle's air vents, having a rear wall provided with inlet air openings, a front wall provided with first outlet air openings, and internal adjustment means , suitable for adjusting the size of the inlet and simultaneously the size of the outlet openings, to adjust the amount of fragrance emitted from the diffuser shell.

Air fresheners are known that use as air freshening element a capsule containing an odorous substance, typically in the form of a gel, the fragrance of which is released into the surrounding area through a semi-permeable microporous membrane of the capsule itself, which is suitable for holding the odorous substance, and at the same time for allowing the aeriform to pass.

Such a capsule is positioned inside its own suitable diffuser, the aeration openings of which allow the air coming out from the vent to hit the microporous membrane to spread the fragrance in the driving compartment.

A particularly notable drawback of this particular type of air freshener for vehicles, however, is the fact that the aforementioned microporous membrane is sensitive to the temperature of the air that hits it, in the sense that by lowering said temperature, the micropores of the membrane tend to shrink.

For this reason, when the temperature of the air is relatively too low, the micropores no longer allow easy passage of the fragrance, the release of which is highly jeopardised.

This drawback is typically evident in summer, when the air conditioning system blows out relatively cold air, thus compromising the efficiency of the air freshener, precisely when there are the conditions that make its use more necessary.

The purpose of the present invention is to overcome the aforementioned drawback of the prior art, with a simple, rational and low-cost solution.

Such a purpose is accomplished through an air freshener for vehicles according to the claims.

Thanks to the invention, when the air coming out from the vent is hot - for example during the winter, when the heating system is operating - the first aeration opening is kept open, so that the air can hit the microporous membrane, obtaining the effect of a better spread of the fragrance inside the driving compartment of the vehicle.

When, on the other hand, the air coming out from the vent is cold - for example during the summer, when the air cooling system is operating - said closing means allow the first aeration opening to be closed, so that the microporous membrane is not hit by the air, avoiding the shrinking of the micropores that would prevent the release of the fragrance. Moreover, thanks to the second aeration opening, said fragrance can still escape the diffuser shell, and be advantageously spread around the driving compartment by the airflow that comes out from the vent, which, whilst not hitting the microporous membrane, licks the diffuser shell.

Further characteristics and advantages of the invention shall become clear from reading the following description provided as a non-limiting example, with the help of the figures illustrated in the attached tables, in which:
- figures 1a, 1b and 1c show perspective views of the same air freshener for vehicles according to the invention, in which the second aeration openings are respectively open, partially open and closed;
- figures 2a and 2b are a view from below of the air freshener of figure 3, in which the first aeration openings are respectively open and closed;
- figure 3 is a side view of the air freshener;
- figure 4 is an exploded view of the air freshener;
- figure 5 is a section along the line V-V indicated in figure 3;
- figure 6 is a side view of an air freshening capsule;

The air freshener 1 for vehicles that is illustrated in the attached figures comprises an air freshening capsule 2 containing an odorous substance S, typically liquid, suitable for releasing its fragrance into the surrounding area, and a diffuser shell 3 that contains said air freshening capsule 2, and is intended to be applied to one of the vents of the vehicle.

The air freshening capsule 2 comprises a generally flat body 20, typically formed from a thin sheet of plastic material - which can without distinction be transparent or opaque, and of any desired colour - that has a convexity 21, generally obtained by thermoforming and possibly - to meet aesthetic requirements - suitably shaped, which defines a chamber 22 suitable for containing said odorous substance S.

Said chamber 22 is closed by a semi-permeable microporous membrane 23 fixed, generally by welding, to the flat perimetric edge of the body 20, which is impermeable to the odorous substance S, preventing it from escaping, and at the same time, permeable to the aeriform, so as to allow the fragrance released by the substance S to go out into the surrounding area.

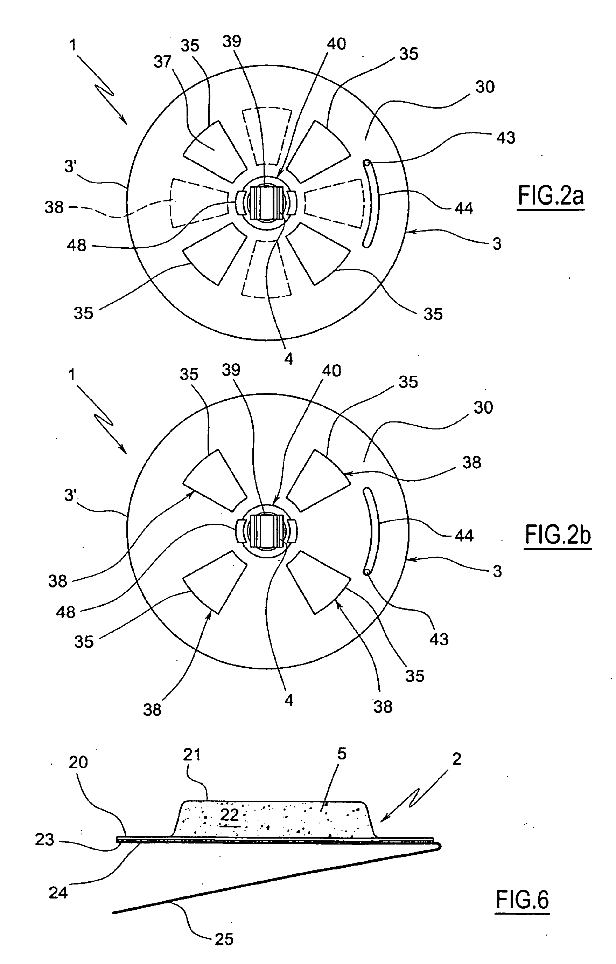

As illustrated in figure 6, the air freshening capsule 2 usually comprises a removable seal 24, having the function of preventing the fragrance from spreading and, therefore, the air freshening effect of the odorous substance S from running out, before the air freshener 1 to which such a capsule 2 belongs is sold. Said removable seal 24 is formed from a second membrane impermeable also to the aeriform, which is stuck onto the first microporous membrane 23, generally by gluing, and a folded tab 25 is foreseen that allows a user to pull it away.

In the air freshener 1 shown, such removal of the seal 24 must take place before the insertion - to be carried out by the user - of the air freshening capsule 2 inside the diffuser shell 3; however, the diffuser shell 3 could be provided with suitable means, for example a side slot, suitable for receiving the folded tab 25 and for making it project outside of the diffuser shell 3, so that the insertion of the air freshening capsule 2 can take place, preferably at the production stage - before the removal of the seal 24.

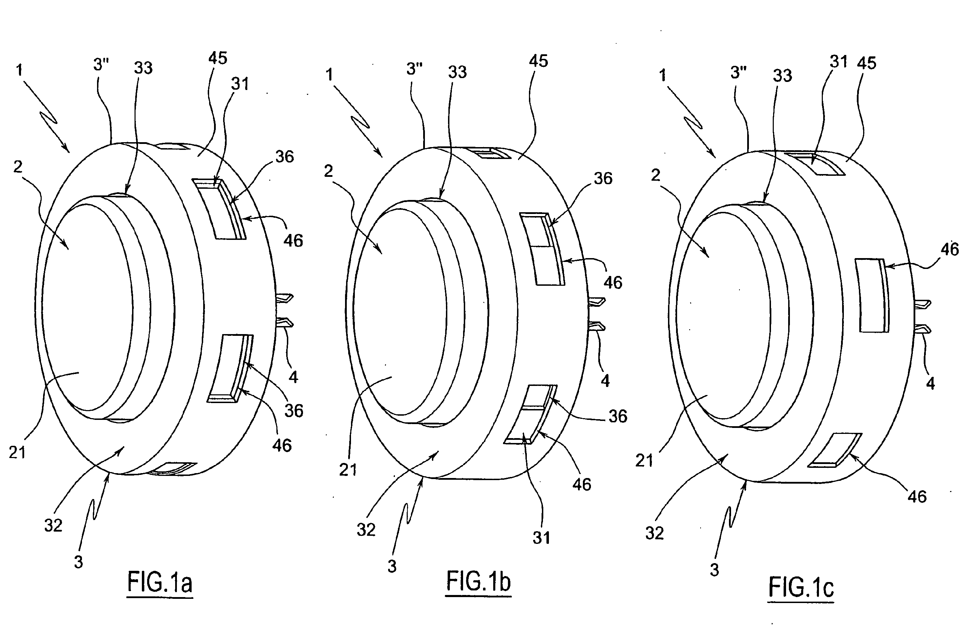

As illustrated in figures 1a to 1c, the diffuser shell 3 is a substantially disc-shaped boxed body, which is provided with an attachment claw 4, projecting from its rear wall 30, suitable for being firmly coupled with an element of the vehicle's air vent.

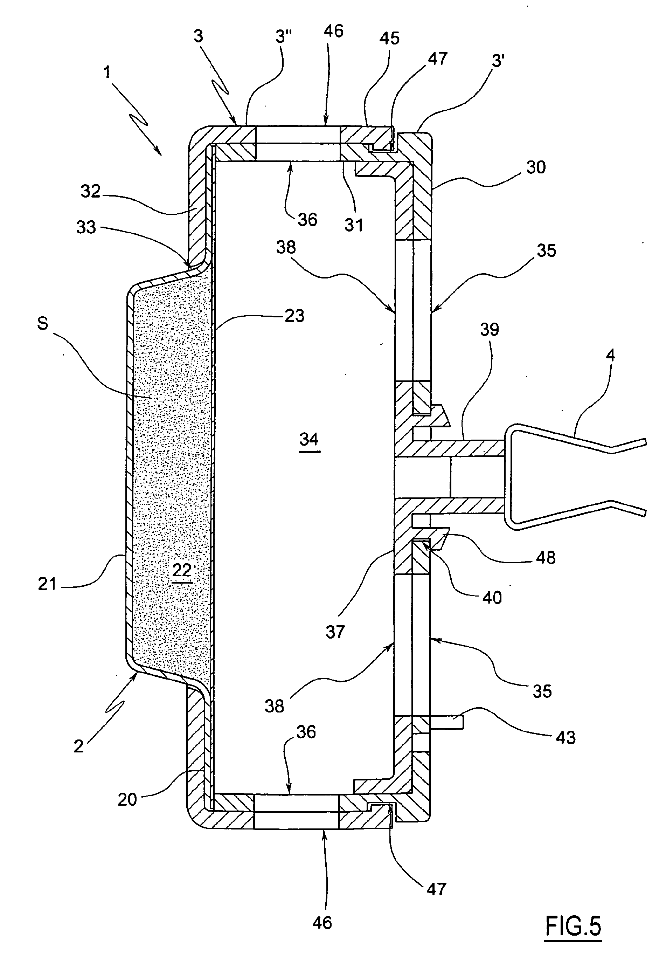

As stated earlier, the diffuser shell 3 is suitable for housing inside it the air freshening capsule 2, which nevertheless does not entirely occupy the available volume, but rather is positioned so that its convexity 21 projects outwards, through a central opening 33 formed in the front wall 32 of the diffuser shell 3 itself. In this way, as can be seen in figure 5, the microporous membrane 23 of the air freshening capsule 2 faces and is distanced from the rear wall 30 of the diffuser shell 3, leaving a large chamber 34 defined, in which the fragrance is collected that, released by the odorous substance S, escapes straight through the microporous membrane 23.

In particular, the diffuser shell 3 comprises two half-shells 3' and 3'', each shaped like cups and with a cylindrical side surface, which are coaxial and inverted with respect to each other, and are rotatably coupled together, so that the respective sides are - for the reasons that shall become clear hereafter - inserted one inside the other. The bottom and the side of the first half-shell 3' respectively define the rear wall 30 and the lateral wall 31 of the diffuser shell 3; whereas the bottom of the second half-shell 3" defines the front wall 32, and is provided with the aforementioned central opening 33.

Between said first and second half-shell 3' and 3" the air freshening capsule 2 is arranged, which closes the concavity of the first half-shell 3' - with the microporous membrane 23 facing towards the rear wall 30 - so as to define the aforementioned collection chamber 34, and is locked in position by the second half-shell 3''; said second half-shell 3" being axially fixed to the first half-shell 3' through a re-entrant ribbing 47 that locks into a corresponding groove formed in the lateral wall 31.

As illustrated in the figures, the diffuser shell 3 is provided with a plurality of aeration openings which place said collection chamber 34 in communication with the outside, including first aeration openings 35 made in the rear wall 30, and second aeration openings 36 made in the lateral wall 31.

In the example shown, in particular, the rear wall 30 has four substantially trapezoidal first aeration openings 35, positioned spoke-like around the axis of the diffuser shell 3 and equally angularly spaced apart; whereas the lateral wall 31 has six substantially rectangular second aeration openings 36, also equally angularly spaced apart with respect to the axis of the diffuser shell 3.

The first aeration openings 35 are suitable for allowing the air coming out from the vent of the vehicle to enter into the collection chamber 34 - since in use the rear wall 30 faces the vent - and to directly hit the microporous membrane 23 of the air freshening capsule 2, to mix with the fragrance released by it and take it along with it, spreading it around the driving compartment.

The second aeration openings 36, on the other hand, are suitable for allowing said fragrance to be released from the collection chamber 34, and are positioned so that the air coming out from the vent cannot, through them, directly hit the microporous membrane 23 of the air freshening capsule 2 - since in use the lateral wall 31 is substantially parallel to the direction in which the air comes out from the vent.

In accordance with the invention, the diffuser shell 3 comprises means suitable for selectively closing the first aeration openings 35, when the temperature of the air coming out from the vent is less than a certain threshold value.

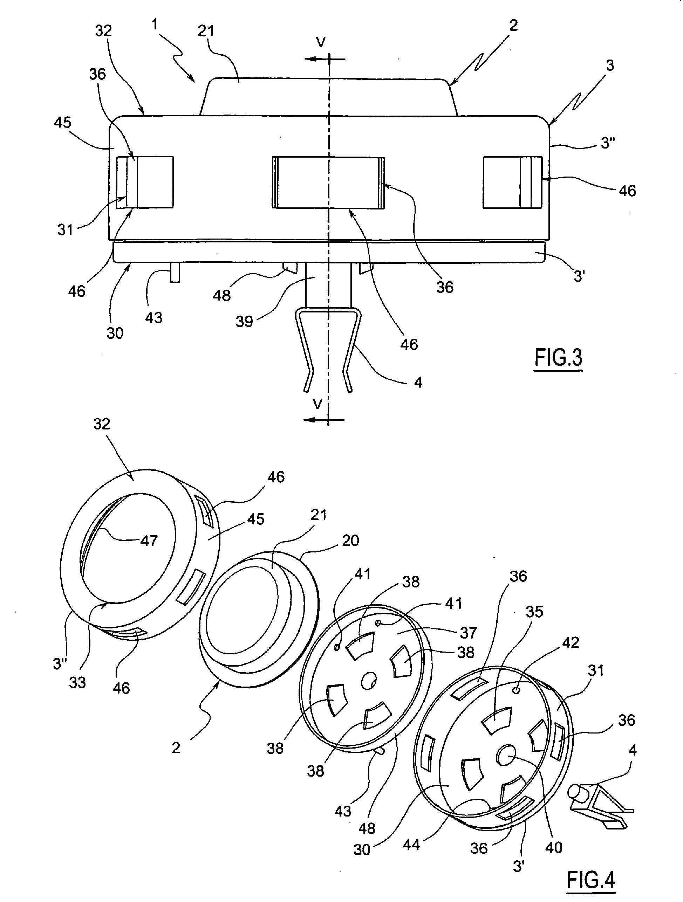

As can be seen in figures 4 and 5, said closing means comprise a first disc-shaped mobile wall 37, arranged coaxially inside the collection chamber 34, substantially facing and in contact with the rear wall 30 of the diffuser shell 3.

Said mobile wall 37 is provided with four passage windows 38, arranged spoke-like around its axis and equally angularly spaced apart, each of which corresponds to a respective first aeration opening 35 of the rear wall 30. In particular, the mobile wall 37 is rotatably fixed to the rear wall 30, and can be positioned in at least two distinct positions with respect to it: a closed position, in which the passage windows 38 are staggered with respect to the first aeration openings 35 that, therefore, are closed by the mobile wall 37 itself; and an open position, in which said passage windows 38 face the first aeration openings 35, so that they are open and allow the passage of the air coming out from the vent.

As illustrated in figure 5, the first mobile wall 37 is rotatably fixed to the rear wall 30 through a pair of flexible projecting tabs 48, having a circular profile in plan, which engage in a corresponding hole 40 of the rear wall 30 itself, and are each equipped with a respective tooth that makes a joint that axially attaches the first mobile wall 37 to the rear wall 30. Moreover, a central pin 39 of the first mobile wall 37, the distal end of which is joined integrally to the aforementioned attachment claws 4, projects axially from the central hole 40.

The diffuser shell 3 comprises suitable means suitable for locking the first mobile wall 37 with respect to the rear wall 30, so as to fix it, when so required, in one of the aforementioned open and closed positions. In the example shown, said locking means comprise two holes 41 formed in the first mobile wall 37, arranged along a circumference having its centre on its axis; and a peg projecting 42 from the rear wall 30, which is suitable for engaging inside one or the other hole 41, according to whether the mobile wall 37 is in open or closed position.

Moreover, means are foreseen suitable for allowing the manual displacement of the mobile wall 37 into the two distinct open and closed positions, which comprise a control pin 43 that, branching out from the mobile wall 37 itself, projects outside of the diffuser shell 3, engaging inside an arched slot 44 formed in the rear wall 30.

In this way, when the temperature of the air coming out from the vent is hot - generally in the winter - the user can arrange the mobile wall 37 in open position, and allow said air to directly hit the microporous membrane 23 of the air freshening capsule 2; whereas when the temperature of the air is cold - generally in summer - he can arrange the mobile wall 37 in closed position and thus avoid the micropores of the membrane 23 being able to shrink to the point of preventing the passage of the fragrance released by the odorous substance S, compromising the effectiveness of the air freshener 1.

It should be emphasised that in this second case, although the air coming out from the vent does not directly hit the air freshening capsule 2, it still remains an effective vehicle for spreading the fragrance; indeed, by licking the second aeration openings 36, the airflow sucks the fragrance, that has in any case been released, out from the collection chamber 34, and mixing with it, spreads it around the driving compartment.

In order to be able to adjust the intensity - in all seasons - of the air freshening produced by the air freshener 1, i.e. the amount of fragrance that can escape from the collection chamber 34, the diffuser shell 3 also comprises adjustment means, distinct and independent from said means for closing the first aeration openings 35, suitable for adjusting the size of the through-port made available by the aforementioned second aeration openings 36.

Said adjustment means generally comprise a second mobile annular wall 45 with circular section, which is coaxially slotted onto the outside of the lateral wall 31 of the diffuser shell 3, so as to be able to slide substantially facing and sticking to it. In particular, in the example shown, said second mobile wall 45 is defined by the side of the second half-shell 51.

The second mobile wall 45 is provided with six passage windows 46, equally angularly spaced apart with respect to the axis of the diffuser shell 3, each of which corresponds to a corresponding second aeration opening 36.

In this way, as illustrated in figures 1a to 1b, the second mobile wall 45 can be engaged to rotate on the lateral wall 31, oscillating between two limit positions: a closed position, in which the passage windows 46 are all staggered with respect to the second aeration openings 36, so that they are closed by the second mobile wall 45 itself; and an open position, in which the passage windows 46 face the second aeration openings 36, and allow the fragrance to escape from the collection chamber 34.

Between said open and closed positions, as can be seen in figure 1b, the second mobile wall 45 can be arranged in any intermediate position, to adjust the amount of fragrance that can come out from the collection chamber 34 as required.

Of course, both for the first mobile wall 37 and for the second mobile wall 45, it is foreseen that the diffuser shell 3 be equipped with suitable means (not shown), suitable for indicating to the user their relative position with regard to the rear wall 30 and the lateral wall 31, respectively.

Of course, numerous practical-application modifications can be made to the invention in object, without for this reason departing from the inventive idea as claimed below.

## Claims

1. Air freshener for vehicles that comprises:
- an air freshening capsule (2) containing an odorous substance (S) that, through a microporous membrane (23) of the air freshening capsule (2) itself, releases a fragrance into the surrounding area; and
- a diffuser shell (3) suitable for enclosing said air freshening capsule (2), which is arranged to be applied to one of the vehicle's air vents, and is provided in a rear wall (30) with at least one first aeration opening (35) facing the microporous membrane (23), through which, in use, the air coming out from the vent goes inside the diffuser shell,
said diffuser shell (3) comprising:
- means (37) for closing said first aeration opening (35); at least
- a second aeration opening (36) suitable for allowing the fragrance to escape from the diffuser shell (3), which is positioned in a lateral wall (31) of the diffuser shell (3) so that, in use, the air coming out from the vent does not hit directly the microporous membrane (23); and
- adjustment means (45), suitable for adjusting the size of the second aeration opening (36), to adjust the amount of fragrance that can escape from the diffuser shell (3), **characterised in that** said adjustment means (45) are distinct and independent from said means (37) for closing the first aeration opening (35),

2. Air freshener according to claim 1, **characterised in that** the diffuser shell (3) comprises a first mobile wall (37) substantially facing and in contact with said rear wall (30), which is provided with at least one passage window (38) and is suitable for being positioned in at least two distinct positions with respect to said rear wall (30): a closed position, in which said passage window (38) is staggered with respect to the first aeration opening (35), and an open position, in which said passage window (38) faces the first aeration opening (35).

3. Air freshener according to claim 2, **characterised in that** said first mobile wall (37) is rotatably attached to the rear wall (30), so as to rotate with respect to it.

4. Air freshener according to claim 2, **characterised in that** the diffuser shell (3) comprises means (41, 42) for locking said first mobile wall (37), with respect to the rear wall (30), in said open and closed positions.

5. Air freshener according to claim 4, **characterised in that** said locking means comprise two holes (41) formed in one of the walls, mobile (37) or rear (30), and a peg (42) fixed to the other, suitable for engaging inside one of said holes (41) according to whether the first mobile wall (37) is in closed or open position.

6. Air freshener according to claim 1, **characterised in that** said lateral wall (31)is substantially parallel to the direction in which the air comes out from the vent.

7. Air freshener according to claim 6, **characterised in that** said adjustment means comprise a second mobile wall (45), provided with at least one passage window (46), which is suitable for sliding substantially facing and sticking to said lateral wall (31), between a closed position, in which said passage window (46) is staggered with respect to the second aeration opening (36), and an open position, in which said passage window (46) faces the second aeration opening (36).

8. Air freshener according to claim 7, **characterised in that** the lateral wall (31) and the second mobile wall (45) are both annular with circular section, and are inserted coaxially inside each other, so that the second mobile wall (45) slides on the lateral wall (31), rotating with respect to it.

9. Air freshener according to claim 2 and 7, **characterised in that** the diffuser shell (3) comprises:
- a first cylindrical cup-shaped half-shell (3'), the bottom of which forms said rear wall (30) and the side wall of which forms said lateral wall (31)
- a second cylindrical cup-shaped half-shell (3''), which is inverted with respect to said first half-shell (3'), and is coaxially and rotatably fixed to it,
the side of said second half-shell being said second mobile wall (45).

## Patentansprüche

1. Duftspender für Fahrzeuge, welcher folgendes aufweist:
- eine Duftspendekapsel (2), welche einen Riechstoff (S) aufweist, der durch eine mikroporöse Membran (23) der Duftspendekapsel (2) selbst einen Duft in die Umgebung freigibt; und
- einen Verteilermantel (3), welcher dafür geeignet ist, die Duftkapsel (2) aufzunehmen, welche so ausgebildet ist, dass sie an einer der Luftdüsen des Fahrzeugs anbringbar ist und welche in einer hinteren Wandung (30) mit wenigstens einer ersten Belüftungsöffnung (35) versehen ist, welche der mikroporösen Membran (23) zugerichtet ist und durch welche bei der Verwendung die aus der Belüftungsdüse austretende Luft in den Verteilermantel strömt,
wobei der Verteilermantel (3) folgendes aufweist:
- eine Einrichtung (37) zum Schließen der ersten Belüftungsöffnung (35); wenigstens
- eine zweite Belüftungsöffnung (36), welche geeignet ist, es dem Duft zu ermöglichen, aus dem Verteilermantel (3) auszutreten, und welche in einer seitlichen Wandung (31) des Verteilermantels (3) so angeordnet ist, dass bei der Verwendung die aus der Belüftungsdüse austretende Luft die mikroporöse Membran (23) nicht direkt trifft; und
- eine Einstelleinrichtung (45), welche dafür geeignet ist, die Größe der zweiten Belüftungsöffnung (36) einzustellen, um die Menge des Duftes, der aus dem Verteilermantel (3) austreten kann, einzustellen,
**dadurch gekennzeichnet , dass**
die Einstelleinrichtung (45) unterschiedlich von und unabhängig von der Einrichtung (37) zum Schließen der ersten Belüftungsöffnung (35) ist.

2. Duftspender nach Anspruch 1,
**dadurch gekennzeichnet , dass**
der Verteilermantel (3) eine erste bewegliche Wandung (37) aufweist, welche im wesentlichen der hinteren Wand (30) zugerichtet und in Kontakt mit derselben ist und welche mit wenigstens einem Durchgangsfenster (38) versehen und in der Lage ist, in wenigstens zwei unterschiedlichen Positionen bezüglich der hinteren Wand (30) angeordnet zu werden: einer geschlossenen Position, in welcher das Durchgangsfenster bezüglich der ersten Belüftungsöffnung (35) versetzt ist, und eine geöffnete Position, in welcher das Durchgangsfenster (38) der ersten Belüftungsöffnung (35) gegenüberliegt.

3. Duftspender nach Anspruch 2,
**dadurch gekennzeichnet , dass**
die erste bewegliche Wand (37) drehbar an der hinteren Wand (30) angebracht ist, um bezüglich derselben zu rotieren.

4. Duftspender nach Anspruch 2,
**dadurch gekennzeichnet , dass**
der Verteilermantel (3) eine Einrichtung (41,42) zum Verriegeln der ersten beweglichen Wand (37) bezüglich der hinteren Wand (30) in der offenen und geschlossenen Position aufweist.

5. Duftspender nach Anspruch 4,
**dadurch gekennzeichnet , dass**
die Verriegelungseinrichtung zwei Bohrungen (41), die in einer der Wände, bewegliche (37) oder hintere (30), gebildet ist, und einen an der anderen angebrachten Stift (42) aufweist, welcher dafür geeignet ist, in eine der Bohrungen (41) in Abhängigkeit davon einzugreifen, ob die erste bewegliche Wand (37) sich in der geschlossenen oder geöffneten Position befindet.

6. Duftspender nach Anspruch 1,
**dadurch gekennzeichnet , dass**
die seitliche Wand (31) im wesentlichen parallel zu der Richtung ist, in welcher die Luft aus der Belüftungsdüse austritt.

7. Duftspender nach Anspruch 6,
**dadurch gekennzeichnet , dass**
die Einstelleinrichtung eine zweite bewegliche Wand (45) aufweist, die mit wenigstens einem Durchgangsfenster (46) versehen ist, welches dafür geeignet ist, im wesentlichen der seitlichen Wand (31) zugerichtet und an derselben anhaftend zwischen einer geschlossenen Position, in welcher das Durchgangsfenster (46) bezüglich der zweiten Belüftungsöffnung (36) versetzt ist, und einer geöffneten Position zu gleiten, in welcher das Durchgangsfenster (46) der zweiten Belüftungsöffnung (36) gegenüberliegt.

8. Duftspender nach Anspruch 7,
**dadurch gekennzeichnet , dass**
die seitliche Wand (31) und die zweite bewegliche Wand (45) beide ringförmig mit einem kreisförmigen Querschnitt und koaxial ineinander eingeführt sind, so dass die zweite bewegliche Wand (45) auf der seitlichen Wand (31) bezüglich derselben rotierend gleitet.

9. Duftspender nach den Ansprüchen 2 und 7,
**dadurch gekennzeichnet , dass**
der Verteilermantel (3) folgendes aufweist:
- eine erste, zylindrische, tassenförmige Halbschale (3'), deren Unterseite die hintere Wand (30) und deren Seitenwand die seitliche Wand (31) bildet,
- eine zweite, zylindrische, tassenförmige Halbschale (3"), welche bezüglich der ersten Halbschale (3') invertiert ist und koaxial zu derselben und drehbar an derselben angebracht ist,
wobei die Seite der zweiten Halbschale die zweite bewegliche Wand (45) ist.

## Revendications

1. Désodorisant pour véhicules comprenant :
- une capsule désodorisante (2) contenant une substance odorante (S) qui, à travers une membrane microporeuse (23) de la capsule désodorisante (2) elle-même, libère un parfum dans la zone environnante ; et
- une coque de diffusion (3) permettant d'enfermer ladite capsule désodorisante (2), qui est disposée pour être appliquée à une des bouches d'air du véhicule, et est prévue dans une paroi arrière (30) avec au moins une première ouverture d'aération (35) en face de la membrane microporeuse (23), à travers laquelle, en cours d'utilisation, l'air sortant de la bouche d'air pénètre dans la coque de diffusion,
ladite coque de diffusion (3) comprenant :
- un moyen (37) destiné à fermer ladite première ouverture d'aération (35) ; au moins
- une deuxième ouverture d'aération (36) permettant au parfum de s'échapper de la coque de diffusion (3), qui est positionnée dans une paroi latérale (31) de la coque de diffusion (3) de sorte que, en cours d'utilisation, l'air sortant de la bouche d'air ne touche pas directement la membrane microporeuse (23) ; et
- des moyens de réglage (45), permettant de régler la dimension de la deuxième ouverture d'aération (36), pour régler la quantité de parfum qui peut s'échapper de la coque de diffusion (3), **caractérisé en ce que** lesdits moyens de réglage (45) sont distincts et indépendants dudit moyen (37) destiné à fermer la première ouverture d'aération (35).

2. Désodorisant selon la revendication 1, **caractérisé en ce que** la coque de diffusion (3) comprend une première paroi mobile (37) sensiblement en face de ladite paroi arrière (30) et en contact avec celle-ci, qui dispose d'au moins une fenêtre de passage (38) et pouvant être positionnée dans au moins deux positions distinctes par rapport à ladite paroi arrière (30) : une position fermée, dans laquelle ladite fenêtre de passage (38) est en quinconce par rapport à la première ouverture d'aération (35), et une position ouverte, dans laquelle ladite fenêtre de passage (38) est en face de la première ouverture d'aération (35).

3. Désodorisant selon la revendication 2, **caractérisé en ce que** ladite première paroi mobile (37) est fixée de manière rotative à la paroi arrière (30), de sorte à pivoter par rapport à celle-ci.

4. Désodorisant selon la revendication 2, **caractérisé en ce que** la coque de diffusion (3) comprend des moyens (41, 42) destinés à verrouiller ladite première paroi mobile (37), par rapport à la paroi arrière (30), dans lesdites positions ouverte et fermée.

5. Désodorisant selon la revendication 4, **caractérisé en ce que** lesdits moyens de verrouillage comprennent deux orifices (41) formés dans une des parois, mobile (37) ou arrière (30), et une cheville (42) fixée à l'autre paroi, pouvant pénétrer dans un desdits orifices (41) selon que la première paroi mobile (37) est en position fermée ou ouverte.

6. Désodorisant selon la revendication 1, **caractérisé en ce que** ladite paroi latérale (31) est sensiblement parallèle à la direction dans laquelle l'air sort de la bouche d'air.

7. Désodorisant selon la revendication 6, **caractérisé en ce que** lesdits moyens de réglage comprennent une deuxième paroi mobile (45), disposant d'au moins une fenêtre de passage (46), pouvant coulisser sensiblement en face de ladite paroi latérale (31) et adhérer à celle-ci, entre une position fermée, dans laquelle ladite fenêtre de passage (46) est en quinconce par rapport à la deuxième ouverture d'aération (36), et une position ouverte, dans laquelle ladite fenêtre de passage (46) est en face de la deuxième ouverture d'aération (36).

8. Désodorisant selon la revendication 7, **caractérisé en ce que** la paroi latérale (31) et la deuxième paroi mobile (45) sont toutes les deux annulaires avec une section circulaire, et sont insérées de manière coaxiale à l'intérieur l'une de l'autre, de sorte que la deuxième paroi mobile (45) coulisse sur la paroi latérale (31), pivotant par rapport à elle.

9. Désodorisant selon les revendications 2 et 7, **caractérisé en ce que** la coque de diffusion (3) comprend :
- une première demi-coque (3') en forme de coupe cylindrique, dont le fond forme ladite paroi arrière (30) et dont la paroi latérale forme ladite paroi latérale (31)
- une deuxième demi-coque (3") en forme de coupe cylindrique, qui est inversée par rapport à ladite première demi-coque (3'), et qui est fixée de manière coaxiale et rotative à celle-ci, le côté de ladite deuxième demi-coque étant ladite deuxième paroi mobile (45).
